(19) Europäisches Patentamt
European Patent Office
Office européen des brevets

(11) Publication number: 0 211 354
A2

(12) # EUROPEAN PATENT APPLICATION

(21) Application number: 86110294.5

(22) Date of filing: 25.07.86

(51) Int. Cl.[4]: C07H 19/073 , A61K 31/33

(30) Priority: 29.07.85 US 760192

(43) Date of publication of application:
25.02.87 Bulletin 87/09

(84) Designated Contracting States:
AT BE CH DE FR GB IT LI LU NL SE

(71) Applicant: MERRELL DOW PHARMACEUTICALS INC.
P.O. Box 156300
Cincinnati Ohio 45215-6300(US)

(72) Inventor: Sunkara, Sai P.
9629 Linfield Drive
Cincinnati Ohio 45242(US)
Inventor: Farr, Robert A.
2960 Maureen Court
Loveland Ohio 45140(US)

(74) Representative: Vossius & Partner
Siebertstrasse 4 P.O. Box 86 07 67
D-8000 München 86(DE)

(54) **Nucleosides and their use as antineoplastic agents.**

(57) This invention relates to the use of certain nucleosides in the treatment of neoplasms, either alone or in conjunctive therapy with ornithine decarboxylase inhibitors and/or S-adenosylmethionine decarboxylase inhibitors.

EP 0 211 354 A2

Xerox Copy Centre

# NUCLEOSIDES AND THEIR USE AS ANTINEOPLASTIC AGENTS

This invention relates to the use of certain nucleosides in the treatment of neoplasms, either alone or in conjunctive therapy with ornithine decarboxylase inhibitors and/or S-adenosylmethionine decarboxylase inhibitors.

More specifically, this invention relates to the treatment of neoplasms with nucleosides of the formula

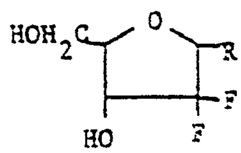

I

or a pharmaceutically acceptable salt thereof wherein R is a base having the structural formulae as follows:

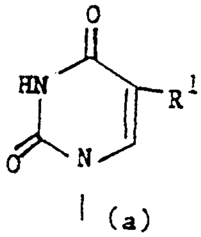
(a)

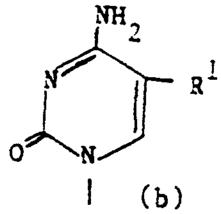
(b)

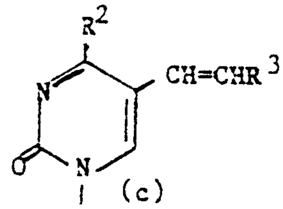
(c)

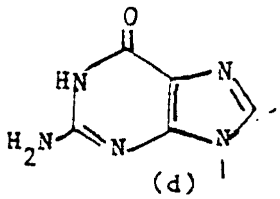
(d)

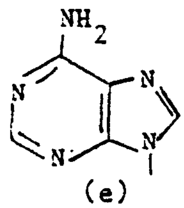
(e)

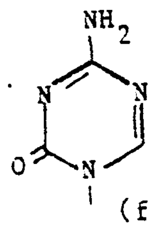
(f)

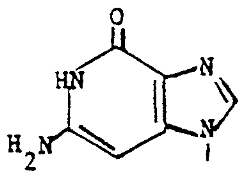
(g)

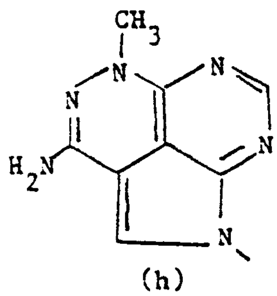
(h)

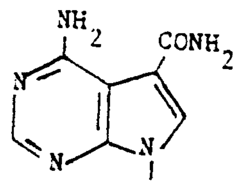
(i)

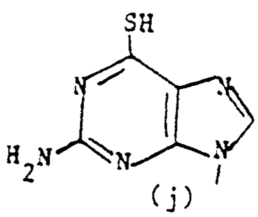
(j)

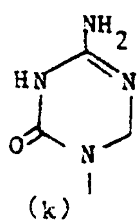
(k)

with $R^1$ being hydrogen, methyl, bromo, fluoro, chloro or iodo,

$R^2$ being hydroxy or amino, and

$R^3$ being chloro, bromo or iodo.

In essence, the depicted compounds of Formula 1 (a-k) are 2-desoxy-2,2-difluorocarbohydrate derivatives bearing the following base moieties:

5-$R^1$-2,4-dioxo-1H,3H-pyrimidin-1-yl (1a),

4-amino-5-$R^1$-2-oxo-1H-pyrimidin-1-yl (1b),

4-$R^2$-5-($R^3$-2-vinyl)-2-oxo-1H-pyrimidin-1-yl (1c),

2-amino-6-oxo-1H,9H-purin-9-yl (1d),

6-amino-9H-purin-9-yl (1e),

4-amino-2-oxo-1H-1,3,5-triazin-1-yl (1f),

6-amino-4-oxo-1,5-dihydro-4H-imidazo[4,5-c]-pyridin-1-yl (1g),

1,5-dihydro-5-methyl-3-amino-1,4,5,6,8-pentaazaacenaphthylen-1-yl (1h),

4-amino-5-carboxamido-7H-pyrrol[2,3-d]pyrimidin-7-yl (1i),

2-amino-6-thione-1,9-dihydro-6H-purin-9-yl (1j),

4-amino-2-oxo-3,6-dihydro-1H,1,3,5-triazin-1-yl (1k), including their tautomeric and enantiomorphic forms and the pharmaceutically acceptable salts thereof.

The above depicted structure does not indicate the stereochemistry of the compounds of the present invention. Compounds of all configurations are believed to be useful, and the stereochemistry of the compound is not to be construed as a limitation. However, the compounds possessing the configuration of naturally occurring ribose (as follows) are preferred:

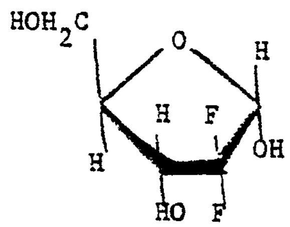

(II)

Further preferred is that the configuration of the juncture between the ribose and the base be as follows:

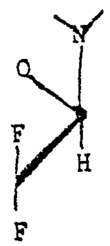

III

Typical of the 2-desoxy-2,2-difluorocarbohydrates embraced within the scope of this invention are:

2-desoxy-2,2-difluororibose,

3,5-bis(trimethylsilyloxy)-2-desoxy-2,2-difluororibose,

3,5-dibenzyloxy-2-desoxy-2,2-difluororibose,

3,5-bis(chloroacetoxy)-2-desoxy-2,2-difluororibose,

3,5-bis(2-chlorobenzyloxy)-1-methanesulfonyloxy-2-desoxy-2,2-difluororibose,

3,5-bis(4-nitrobenzyloxy)-1-(4-toluenesulfonyloxy)-2-desoxy-2,2-difluororibose,

1-chloro-3,5-bis(phenoxyacetoxyl)-2-desoxy-2,2-difluoroxylose,

1-(2,4-dibromophenylsulfonyloxy)-3,5-bis(2,2-dimethylpropionyloxy)-2-desoxy-2,2-difluoroxylose,

3,5-bis(benzoyloxy)-1-(o-toluenesulfonyloxy)-2-desoxy-2,2-difluoroxylose,

1-bromo-3,5-bis(methoxycarbonyloxy)-2-desoxy-2,2-difluoroxylose,

3,5-bis(allyloxycarbonyloxy)-1-chloro-2-desoxy-2,2-difluoroxylose,

3,5-bis(benzyloxycarbonyloxy)-2-desoxy-2,2-difluoroxylose,

1-bromo-3,5-bis(4-nitrobenzyloxycarbonyloxy)-2-desoxy-2,2-difluoroxylose,

1-bromo-3,5-bis(tetrahydrothienyloxy)-2-desoxy-2,2-difluororibose,

1-bromo-3,5-bis(isopropyldimethylsilyloxy)-2-desoxy-2,2-difluororibose,

1-(2-chlorophenylsulfonyloxy)-3,5-bis-(methoxymethoxy)-2-desoxy-2,2-difluororibose,

3,5-bis(benzyloxymethoxy)-2-desoxy-2,2-difluororibose,

1-(4-nitrophenylsulfonyloxy)-3,5-bis(trityloxy)-2-desoxy-2,2-difluororibose,

3,5-bis(allyloxy)-1-chloro-2-desoxy-2,2-difluororibose,

2-desoxy-2,2-difluoroxylose,

3,5-bis(trimethylsilyloxy)-2-desoxy-2,2-difluoroxylose,

3,5-dibenzyloxy-2-desoxy-2,2-difluoroxylose,

3,5-bis(chloroacetoxy)-2-desoxy-2,2-difluoroxylose,

3,5-bis(2-chlorobenzyloxy)-1-methanesulfonyloxy-2-desoxy-2,2-difluoroxylose,

3,5-bis(4-nitrobenzyloxy)-1-(4-toluenesulfonyloxy)-2-desoxy-2,2-difluoroxylose,

1-bromo-3,5-bis(tetrahydrothienyloxy)-2-desoxy,2,2-difluoroxylose,

1-bromo-3,5-bis(isopropyldimethylsilyloxy)-2-desoxy-2,2-difluoroxylose,

3,5-bis( -butyldiphenylsilyloxy)-2-desoxy-2,2-difluororibose,

3,5-bis(formyloxy)-1-isopropylsulfonyloxy-2-desoxy,2,2-difluororibose,

3,5-bis(trichloroacetoxy)-1-methanesulfonyloxy-2-desoxy-2,2-difluororibose,

1-chloro-3,5-bis(phenoxyacetoxy)-2-desoxy-2,2-difluororibose,

1-(2,4-dibromophenylsulfonyloxy)-3,5-bis(2,2-dimethylpropionyloxy)-2-desoxy-2,2-difluororibose,

3,5-bis(benzoyloxy)-1-(o-toluenesulfonyloxy)-2-desoxy-2,2-difluororibose,

1-bromo-3,5-bis(methoxycarbonyloxy)-2-desoxy-2,2-difluororibose,

1-(2-chorophenylsulfonyloxy)-3,5-bis-(methoxymethoxy)-2-desoxy-2,2-difluoroxylose,

3,5-bis(benzyloxymethoxy)-2-desoxy-2,2-difluoroxylose,

1-(4-nitrophenylsulfonyloxy)-3,5-bis(trityloxy)-2-desoxy-2,2-difluoroxylose,

3,5-bis(allyloxy)-1-chloro-2-desoxy-2,2-difluoroxylose,

3,5-bis( *t*-butyldiphenylsilyloxy)-2-desoxy-2,2-difluoroxylose,

3,5-bis(formyloxy)-1-isopropylsulfonyloxy-2-desoxy-2,2-difluoroxylose,

3,5-bis(trichloroacetoxy)-1-methanesulfonyloxy-2-desoxy-2,2-difluoroxylose,

3,5-bis(allyloxycarbonyloxy)-1-chloro-2-desoxy-2,2-difluororibose,

3,5-bis(benzyloxycarbonyloxy)-2-desoxy-2,2-difluororibose,

1-bromo-3,5-bis(4-nitrobenzyloxycarbonyloxy)-2-desoxy-2,2-difluororibose,

One skilled in the art would be aware of the bases which are used in the synthesis of the antitumor nucleosides of the present invention, but the following nucleosides are given to further elaborate the type of antitumor agents which this invention makes available.

1-(5-methyl-2,4-dioxo-1H,3H-pyrimidin-1-yl)-2-desoxy-2,2-difluororibose,

1-(2,4-dioxo-1H,3H-pyrimidin-1-yl)-2-desoxy-2,2-difluororibose,

1-(5-bromo-2,4-dioxo-1H,3H-pyrimidin-1-yl)-2-desoxy-2,2-difluororibose,

1-(5-chloro-2,4-dioxo-1H,3H-pyrimidin-1-yl)-2-desoxy-2,2-difluororibose,

1-(5-iodo-2,4-dioxo-1H,3H-pyrimidin-1-yl)-2-desoxy-2,2-difluororibose,

1-(4-amino-5-chloro-2-oxo-1H-pyrimidin-1-yl)-2-desoxy-2,2-difluororibose,

1-(4-amino-5-bromo-2-oxo-1H-pyrimidin-1-yl)-2-desoxy-2,2-difluororibose,

1-(4-amino-2-oxo-1H-pyrimidin-1-yl)-2-desoxy-2,2-difluororibose,

1-(4-amino-5-iodo-2-oxo-1H-pyrimidin-1-yl)-2-desoxy-2,2-difluororibose,

1-(4-amino-5-methyl-2-oxo-1H-pyrimidin-1-yl)-2-desoxy-2,2-difluororibose,

1-[5-(2-bromovinyl)-4-hydroxy-2-oxo-1H-pyrimidin-1-yl]-2-desoxy-2,2-difluororibose,

1-[4-amino-5-(2-bromovinyl)-2-oxo-1H-pyrimidin-1-yl]-2-desoxy-2,2-difluororibose,

1-[4-amino-5-(2-iodovinyl)-2-oxo-1H-pyrimidin-1-yl]-2-desoxy-2,2-difluororibose,

1-[5-(2-chlorovinyl)-4-hydroxy-2-oxo-1H-pyrimidin-1-yl]-2-desoxy-2,2-difluororibose,

1-[4-hydroxy-5-(2-iodovinyl)-2-oxo-1H-pyrimidin-1-yl]-2-desoxy-2,2-difluororibose,

1-[4-amino-5-(2-chlorovinyl)-2-oxo-1H-pyrimidin-1-yl]-2-desoxy-2,2-difluororibose,

1-(2-amino-6-oxo-1H,9H-purin-9-yl)-2-desoxy-2,2-difluororibose,

1-(6-amino-9H-purin-9-yl)-2-desoxy-2,2-difluororibose,

1-(4-amino-2-oxo-1H-1,3,5-triazin-1-yl)-2-desoxy-2,2-difluororibose,

1-(6-amino-4-oxo-4H-imidazo[4,5-c]pyridin-1-yl)-2-desoxy-2,2-difluororibose,

1-(3-amino-5-methyl-1,4,5,6,8-pentaazaacenaphthylen-1-yl)-2-desoxy-2,2-difluororibose,

1-(4-amino-5-carboxamido-7H-pyrrolo[2,3-d]pyrimidin-7-yl)-2-desoxy-2,2-difluororibose,

1-(2-amino-6-thione-1,9-dihydro-6H-purin-9-yl)-2-desoxy-2,2-difluororibose,

1-(4-amino-2-oxo-3,6-dihydro-1 H-1,3,5-triazin-1-yl)-2-desoxy-2,2-difluororibose,

1-(5-iodo-2,4-dioxo-1H,3H-pyrimidin-1-yl)-2-desoxy-2,2-difluoroxylose,

1-(2,4-dioxo-1H,3H-pyrimidin-1-yl)-2-desoxy-2,2-difluoroxylose,

1-(5-bromo-2,4-dioxo-1H,3H-pyrimidin-1-yl)-2-desoxy-2,2-difluoroxylose,

1-(5-chloro-2,4-dioxo-1H,3H-pyrimidin-1-yl)-2-desoxy-2,2-difluoroxylose,

1-(5-iodo-2,4-dioxo-1H,3H-pyrimidin-1-yl)-2-desoxy-2,2-difluoroxylose,

1-(4-amino-5-fluoro-2-oxo-1H-pyrimidin-1-yl)-2-desoxy-2,2-difluororibose,

1-(4-amino-5-chloro-2-oxo-1H-pyrimidin-1-yl)-2-desoxy-2,2-difluoroxylose,

1-(4-amino-2-oxo-1H-pyrimidin-1-yl)-2-desoxy-2,2-difluoroxylose,

1-(4-amino-5-fluoro-2-oxo-1H-pyrimidin-1-yl)-2-desoxy-2,2-difluoroxylose,

1-(4-amino-5-methyl-2-oxo-1H-pyrimidin-1-yl)-2-desoxy-2,2-difluoroxylose,

1-[5-(2-bromovinyl)-4-hydroxy-2-oxo-1H-pyrimidin-1-yl]-2-desoxy-2,2-difluoroxylose,

1-[4-amino-5-(2-bromovinyl)-2-oxo-1H-pyrimidin-1-yl]-2-desoxy-2,2-difluoroxylose,

1-[4-amino-5-(2-iodovinyl)-2-oxo-1H-pyrimidin-1-yl]-2-desoxy-2,2-difluoroxylose,

1-[5-(2-chlorovinyl)-4-hydroxy-2-oxo-1H-pyrimidin-1-yl]-2-desoxy-2,2-difluoroxylose,

1-[4-hydroxy-5-(2-iodovinyl)-2-oxo-1H-pyrimidin-1-yl]-2-desoxy-2,2-difluoroxylose,

1-[4-amino-5-(2-chlorovinyl)-2-oxo-1H-pyrimidin-1-yl]-2-desoxy-2,2-difluoroxylose,

1-(2-amino-6-oxo-1H,9H-purin-9-yl)-2-desoxy-2,2-difluoroxylose,

1-(6-amino-9H-purin-9-yl)-2-desoxy-2,2-difluoroxylose,

1-(4-amino-2-oxo-1H-1,3,5-triazin-1-yl)-2-desoxy-2,2-difluorooxylose,

1-(6-amino-4-oxo-4H-imidazo[4,5-c]pyridin-1-yl)-2-desoxy-2,2-difluorooxylose,

1-(3-amino-5-methyl-1,4,5,6,8-pentaazaacenaphthylen-1-yl)-2-desoxy-2,2-difluorooxylose,

1-(4-amino-5-carboxamido-7H-pyrrolo[2,3-d]pyrimidin-7-yl)-2-desoxy-2,2-difluorooxylose,

1-(2-amino-6-thione-1,9-dihydro-6H-purin-9-yl)-2-desoxy-2,2-difluorooxylose,

1-(4-amino-2-oxo-3,6-dihydro-1H-1,3,5-triazin-1-yl)-2-desoxy-2,2-difluorooxylose, or the pharmaceutically acceptable satls thereof.

The preferred compounds of formula 1 are those nucleosides wherein R is 5-fluoro-2,4-dioxo-1H,3H-pyrimidin-1-yl (1a, $R^1 = F$),

4-amino-2-oxo-1H-1,3,5-triazin-1-yl (1f), 4-amino-5-fluoro-2-oxo-1H-pyrimidin-1-yl (1b, $R^1 = F$), and 5-(2-bromovinyl)-4-hydroxy-2-oxo-1H-pyrimidin-1-yl - (1c, $R^2 = OH$, $R^3 = Br$). Most preferred is the compound 2'-desoxy-2',2'-difluorocytidine.

The compounds are believed to exert the antineoplastic effect by virtue of their interruption of the nucleoside metabolism involved in tumor growth. Thus, the compounds are expected to be particularly useful in the treatment of such neoplasms as leukemia, including but not limited to acute lymphoblastic, chronic lymphocytic, acute myloblastic and chronic mylocytic; Carcinomas, including but not limited to those of the cervix, oesphagus, stomach, small intestines, colon and lungs; Sarcomas, including but not limited to oesteroma, osteosarcoma, lepoma, liposarcoma, hemangioma and hemangiosarcoma; Adenomas, both benign and malignant, including, for example, the thyroid, parathyroid, prostate and the like; Melanomas, including amelonotic and melanotic; and mixed types of neoplasias such as, for example, adenocarcinoma, carcinosarcoma, lymphoid tissue type, folicullar reticulum, cell sarcoma and Hodgkins Disease. In effecting treatment of the neoplastic disease state, it is preferred to administer a compound of formula I to a patient suffering from a neoplasm with about 1-50 mg per kilogram of body weight per day. The specific amount depends upon the severity of the state of the neoplasm such as may be determined by the attending physician. In practice, the ideal is to deprive the diseased cell of its opportunity for proliferative cell growth by interrupting nucleoside metabolism. Thus, in the practice of this invention, it is preferred that a sufficient blood level of the nucleotide of formula I be maintained so as to inhibit nucleoside metabolism. The precise dosage required to maintain the desired blood level of a nucleoside of formula I to effect treatment of tumors may be determined by the use of standard pharmaco-kinetic studies well established for these determinations. Of course the compounds of this invention may be administered to a patient orally, intravenously, intraperitoneally and/or subcutaneously, preferably orally. In any case, the pharmaceutical preparations may be effected by standard techniques well known in the art.

The term "treatment of neoplasms" is meant to include treating the patient having neoplasms so that the therapy has an advantageous effect by "controlling the growth" of the neoplasm, which for the purposes of this invention includes slowing, interrupting, arresting or stopping the growth and metastases of the proliferating tumor tissue in a warm-blooded animal; it being understood that such does not necessarily provide a "cure" for the tumor in the sense that the tumor tissue is totally eliminated.

Another aspect of this invention is to utilize the compounds of this invention with ornithine decarboxylase inhibitors (ODC inhibitors) and/or S-adenosylmethione decarboxylase inhibitors (SAMDC inhibitors). Although any of the functional irreversible ODC inhibitors may be utilized in conjunctive therapy with the compounds of formula I, compounds such as methyl acetylenic putrescine and compounds of the formulae

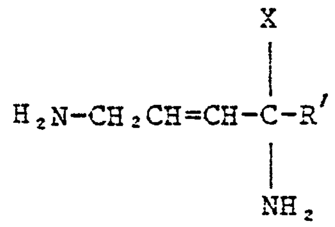

and

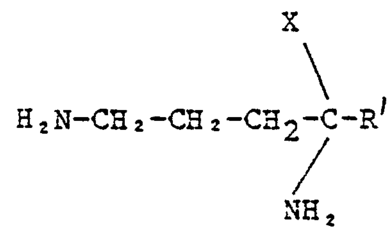

wherein X is $-CHF_2$ or $-CH_2F$,

R'is H or COR",

R" is OH or C $_{1-6}$ lower alkoxy,

and the pharmaceutically acceptable salts and individual optical isomers thereof are preferred. Particularly preferred compounds are α-difluoromethyl ornithine (α-DFMO), the methyl and ethyl esters of monofluoromethyl dehydroornithine, and the R,R-isomer of methyl acetylenic putrescine (i.e., (2R, 5R)-6-heptyne-2, 5-diamine.

In a preferred manner, a compound of this invention can be administered with an ODC or SAMDC inhibitor. When such combination therapy is employed for the treatment of a tumor, the nucleoside (I) may be administered at the above stated dosage to be effective alone. However, the combination with an ODC or SAMDC inhibitor may produce an additive or synergistic effect with the compounds of formula I. Thus, when such combination antitumor therapy is used, the dosage of the nucleoside agent administered may be less than that administered when the nucleoside is used alone. In combination with an ODC inhibitor, the nucleoside compounds of this invention may, therefore, be administered at a lower dosage level or at less frequent intervals as compared to the nucleoside when used alone.

The term "combination therapy" or "conjunctive therapy" contemplates the administration of an ODC or SAMDC inhibitor immediately prior to the beginning of therapy with a nucleoside of this invention concommitantly with the nucleoside, or during the period of time immediately following cessation of such ODC or SAMDC therapy. Preferably, the patient is treated with an ODC inhibitor for about 1 to 14 days, preferably 4 to 14 days, prior to the beginning of therapy with a nucleoside of this invention, and thereafter, on a daily basis during the course of such therapy. Daily treatment with the ODC inhibitor can be continued for a period of, for example, 1 to 365 days after the last dose of the nucleoside is administered.

As pharmacologically useful agents, the compounds of this invention can be administered in various ways to the patient being treated to achieve the desired effect. The compounds can be administered either alone or in combination with one another, or they can be administered with the other aforementioned compounds of this invention in the form of pharmaceutical compositions, the preparation of which are well known in the art. The compounds may be administered orally or parenterally (for example, intravenously, intraperitoneally or subcutaneously), including injection of the active nucleosides directly into the tumor. The amount of compound administered will vary over a wide range and can be any effective amount. Depending upon the patient to be treated, the severity of the condition being treated, the mode of administration, and the particular compound employed, the effective amount of compound of this invention (I) administered will vary from about 1 mg/kg to 50 mg/kg of body weight of the patient per day and preferably will be about 1 mg/kg to 10 mg/kg of body weight of patient per day.

The solid unit dosage forms can be of the conventional type. Thus, the solid form can be a capsule which can be of the ordinary gelatin type containing a novel compounds of this invention and a carrier, for example, lubricant and inert fillers, such as lactose, sucrose and corn starch. In another embodiment, the novel compounds are tableted with conventional tablet bases such as lactose, sucrose or corn starch in combination with binders such as acacia, corn starch or gelatin, disintegrating agents such as corn starch, potato starch or alginic acid, and a lubricant such as stearic acid or magnesium stearate.

For parenteral administration the compounds may be administered as injectable dosages of a solution of suspension of the compound in a physiologically acceptable diluent with a pharmaceutical carrier which can be a sterile liquid such as water and oils with or without the addition of a surfactant and other pharmaceutically acceptable or adjuvants. Illustrative of oils which can be employed in these preparations are those of petroleum, animal, vegetable or synthetic orgini, for example, peanut oil, soybean oil and mineral oil. In general, water, saline, aqueous dextrose and related sugar solutions ethanols and glycols such as propylene glycol or polyethylene glycol are preferred liquid carriers, particularly for injectable solutions.

The results in the treatment of rapidly proliferating tumor tissue disease states with nucleosides of this invention have demonstrated a striking tumor supression and such results can be shown and ascertained by standard and well-known laboratory techniques as may be illustrated by the following.

## TABLE I

Effect of 2'-desoxy -2'2'-difluorocytidine (MDL 27,069) On the Growth of Hela Cells in Culture

| Treatment (μg/ml) | Cell Number ($X10^5$) | % Inhibition |
|---|---|---|
| Control | 3.87 | --- |
| MDL 27,069 | | |
| 0.01 | 0* | 100 |
| 0.005 | 0.36 | 91 |
| 0.0025 | 0.51 | 87 |
| 0.00125 | 1.17 | 70 |
| 0.00062 | 2.0 | 48 |

Hela cells (1 x $10^5$/35 mm dish) were plated and incubated at 37°C in a $CO_2$ (5%) incubator. Media containing different dilutions of MDL 27,069 were added after overnight incubation. At the end of 72 hours, the cells were collected by trypsinization and counted using a coulter counter.

*All cells were killed.

TABLE II

Antitumor activity of 2'-desoxy-2',2'-difluorocytidine against L1210 mouse leukemia.

| TREATMENT | MEAN SURVIVAL TIME (DAYS) | CURES | o/o T/C |
|---|---|---|---|
| Control, | 7.0 | 0/5 | |
| 2'-desoxy-2',2'-difluoro-cytidine | | | |
| 0,01 mg/kg | 13.25 | 0/5 | 189 |
| 0.25 mg/kg | 13.40 | 0/5 | 191 |
| 0.5 mg/kg | 17.40 | 0/5 | 249 |
| 1.0 mg/kg | 20.0 | 2/5* | 286 |

$1 \times 10^5$ L1210 leukemia cells per animal were injected i.p. on day 0. Treatment with the drug was started on day 1. The compound was dissolved in sterile water and administered i.p. at the doses indicated from day 1 to day 9 once daily. Therapeutic effect of the compound was determined by determining the survival time of the treated group in comparison with control group as follows:

$$o/o\ T/C = \frac{\text{survival time in treated group}}{\text{survival time in control group}} \times 100$$

*Animals surviving for more than 30 days are considered cured.

In part, the compounds of formula I are known compounds, wherein the structures and the methods for their preparation are fully elucidated in British patent application 2,136,425A, published September 19, 1984. Those compounds which are novel (i.e., compounds 1 f, g, h, i, j, k) may readily be prepared by analogous techniques taught in the aforementioned British application and by techniques well known in the art. For convenience, the teachings of the aforementioned British patent application are repeated here.

The preparation of the 2-desoxy-2,2-difluorocarbohydrates useful in the coupling reaction are prepared by reacting a D-glyceraldehyde ketonide of the formula

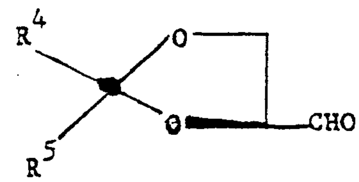

(V)

in which $R^4$ and $R^5$ are, independently, $C_{1-3}$ alkyl, with a $C_{1-4}$ alkyl bromodifluoroacetate, preferring the ethyl ester.

The preferred glyceraldehyde ketonide is the acetonide in which $R^4$ and $R^5$ are both methyl (see Fischer and Baer, Helv. Chim. Acta. 17,622 (1934). Ethyl bromodifluoroacetate was prepared first by Morel and Dawans, Tet. 33, 1445(1977). The reaction of the ketonide and the haloacetate is carried out in the presence of an activated metal such as magnesium or preferably zinc. Activation is obtained most easily by applying ultrasonic energy to the reaction mixture. Activation by that means compensates for the presence of a small amount of water in the reaction mixture, avoiding the necessity to maintain anhydrous conditions, and also avoids the necessity to prepare and carefully store activated metals. However, if desired, the metal may be activated by the customary methods known in the art. Approximately an equimolar amount of metal is the most advantageous amount.

The reaction is performed in ethers such as tetrahydrofuran and diethyl ether, at moderate temperatures. However, other organic solvents which are inert to the reaction conditions may be used, including halogenated alkanes such as chloroform, dichloromethane, or trichloromethane, and aromatic solvents including benzene, toluene and the xylenes. Temperatures in the range of from about ambient temperature to about 150° may be used; temperatures from about ambient temperature to about 80° are preferred, however. Economically acceptable yields have been obtained in reaction times ranging from a few minutes to a few hours. One should note that the reaction is exothermic, and the mixture may need to be cooled, not heated, depending on the scale of the reaction and the rate at which the reactants are added.

The product of the first reaction is an alkyl 3-dioxolanyl-2,2-difluoro-3-hydroxypropionate of formula (IV):

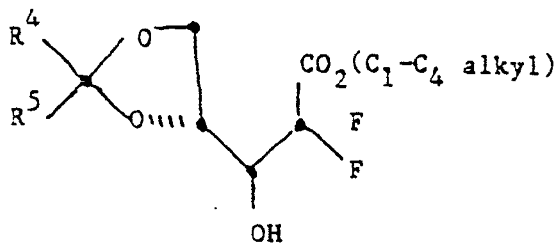

(IV)

in which $R^4$ and $R^5$ are as described earlier.

The ratio of the 3-R-hydroxy intermediate to its 3-S-hydroxy enantiomer is usually about 3:1. The 3-R-hydroxy enantiomer has the proper stereochemistry to produce the ribose derivative in its natural configuration, and so it is the desired enantiomeric product of the first step. The 3-R-hydroxy enantiomer can be separated cleanly from the 3-S-enantiomer by chromatography on silica gel, eluting with chloroform containing 0.5% methanol.

The hydroxypropionate, in either form, is hydrolyzed using very mild conditions to form the lactone of formula (III):

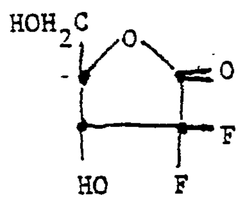

(III)

Proper control of the hydrolysis step will cleave the ketonide function and, unexpectedly, will also cleave the ester group, providing the lactone in a single step. The hydrolysis reagent preferably is a mildly acidic ion exchange resin, of which Dowex 50W---X12 (Dow Chemical Company) is most highly preferred. Carrying out the process with other mild hydrolytic reagents is possible although larger amount of by-products may be obtained. For example, aqueous acetic acid, or other relatively strong acids such as propionic acid, formic acid, chloroacetic acid, or oxalic acid may be used for the hydrolysis.

The hydroxy groups of the lactone should be protected before its keto oxygen is reduced. The usual reaction conditions are used, depending on the protecting groups chosen. For example the *t*-butyldimethylsilyl group is most conveniently provided in the form of its trifluoromethanesulfonate, and the protection reaction is carried out in the presence of a base such as lutidine, pyridine and the like. Acyl protecting groups such as acetyl, benzoyl and the like are provided by reacting the lactone with an acylating agent such as acyl chloride, bromide, cyanide or azide, or with an appropriate anhydride. The reactions are conveniently carried out in a basic solvent such as pyridine, quinoline or isoquinoline, or in a tertiary amine solvent such as triethylamine, tributylamine, or methylpiperidine. The reaction also may be carried out in an inert solvent, to which an acid scavenger, such as a tertiary amine, has been added. Acylation catalysts such as 4-dimethylaminopyridine or 4-pyrrolidinopyridine may be used in the reaction, if desired. The acylation reactions which provide protecting groups on the hydroxy groups are carried out at moderate temperatures in the range of from -25° to 100°. Such acylations also may be performed by acid-catalyzed reactions of the appropriate carboxylic acids, in inert organic solvents or performed by acid-catalyzed reactions of the appropriate carboxylic acids, in inert organic solvents or neat. Acid catalysts such as sulfuric acid, polyphosphoric acid, or methanesulfonic acid may be used.

Acyl protecting groups may also be provided by forming an active ester of the appropriate acid, such as the esters formed by such known reagents as dicyclohexylcarbodiimide, acylimidazoles, nitrophenols, pentachlorophenol, N-hydroxysuccinimide and 1-hydroxybenzotriazole.

Protecting groups of the ether type are produced by reacting the lactone with, for example, an appropriate diazo compound, such as diazomethane, phenyldiazomethane or a silyldiazomethane. Such reactions commonly are carried out in solvents including esters such as ethyl acetate, halogenated solvents including dichloromethane and chloroform, and ethers including diethyl ether and tetrahydrofuran. The process is usually carried out at low temperatures from about -50° to about 0°. Such ether-forming reactions may also be carried out with the assistance of reagents such as trimethyloxosulfonium hydroxide, trimethylsulfonium hydroxide and trimethylselenonium hydroxide, in solvents such as dimethylsulfoxide, dimethylformamide, hexamethylphosphoramide, acetone, or acetonitrile.

The silyl protecting groups discussed above are placed on the hydroxy groups by the conventional methods, such as by reaction with the appropriate silylcarboxamide or bis(substituted-silyl)-carboxamide, or an appropriately substituted silazane. Suitably substituted silyl methanesulfonates, toluenesulfonates and the like are useful also. An equivalent amount of a base is usually necessary in the reaction mixture, unless a basic solvent such as is discussed above is used in the reaction.

When the hydroxy groups have been protected, the keto oxygen of the lactone is reduced to the alcohol, forming the protected 2-desoxy-2,2-difluororibose or xylose of this invention. The most preferred reducing agent is diisobutylaluminum hydride, used at a low temperature in the range of about -100° to -20°. The reduction must be performed very carefully to avoid conditions so vigorous that the ring is opened at the oxygen atom. Other metal hydrides, such as the widely used lithium aluminum hydride, can also be used for the reduction, but it is necessary to keep the temperature quite low and to assure that the hydride is destroyed before the temperature is allowed to rise

toward ambient. Accordingly, a solvent with a very low freezing point must be used in the reduction step. Toluene is convenient; other solvents, of course, can be used, especially ethers such as diethyl ether.

To obtain efficient reaction with the base, an appropriate leaving group must be placed at the 1-position of the carbohydrate. The preferred leaving group is methanesulfonyl, which is readily provided by reaction with methanesulfonyl chloride in the presence of an equivalent amount of a suitable acid scavenger such as triethylamine and the like. Other sulfonyl leaving groups are provided in the same way by reaction with the appropriate sulfonyl halide.

When a chloro or bromo leaving group is to be used, it is frequently advantageous first to make the 1-acetate derivative, as by reaction with acetic anhydride, or another source of acetyl groups, in the presence of an equivalent amount or more of an acid scavenger. The acetate group then is displaced, at a low temperature such as about -50° to about 0°, with gaseous hydrogen bromide or hydrogen chloride. Because the gaseous hydrogen halide may tend to remove the protecting groups, especially silyl protecting groups, operating this step at low temperatures and adding the hydrogen halide slowly in small increments is necessary.

The coupling reactions with the bases (1 a-k) and the 2-desoxy-2,2-difluorocarbohydrates to obtain the products of formula I are of such a nature that hydroxy groups must be protected to keep them from reacting with other reagents present in the reaction. The protecting groups are those commonly used in synthetic organic chemistry. Chemists are accustomed to choosing groups which can be placed efficiently on hydroxy groups and which can be removed easily when the reaction is complete. Suitable groups may be those described in standard textbooks such as Chapter 3 of Protective Groups in Organic Chemistry, McOmie, Ed., Plenum Press, New York (1973); and Chapter 2 of Protective Groups in Organic Synthesis, Greene, John Wiley & Sons, New York (1981).

For example, hydroxy-protecting groups may include the formyl, 2-chloroacetyl, benzyl, diphenylmethyl, triphenylmethyl, 4-nitrobenzyl, 2-methoxyethoxymethyl, methoxyacetyl, phenoxyacetyl, tetrahydropyranyl, allyl, tetrahydrothienyl, isobutyryl, ethoxycarbonyl, or benzyloxycarbonyl groups. Silyl hydroxy-protecting groups are particularly convenient because most of them are cleaved easily by contact with water or an alcohol. Such groups may include especially trimethylsilyl, as well as isopropyldimethylsilyl, methyldiisopropylsilyl, or triisopropylsily. The *t*-butyldimethylsilyl group is a special case and is preferred as the protecting group in this synthesis: it is more difficult to cleave, requiring a reagent such as a hydrohalic acid to remove it from the hydroxy groups.

Ribose and xylose have a hydroxy group at the 1-position of its ring. In order to react the carbohydrate of this invention with the base, to form the antitumor compounds of this invention, a leaving group must be placed at the 1-position. The leaving groups are those typically used in organic synthesis. The preferred leaving groups are sulfonates, of which the most preferred is methanesulfonate: other typical leaving groups such as toluenesulfonate, ethanesulfonate, isopropanesulfonate, 4-methoxybenzenesulfonate, 4-nitrobenzenesulfonate, 2-chlorobenzenesulfonate, chloro and bromo also may be used.

The bases used to form the antitumor compounds of the present invention are known to those skilled in the art, and no discussion of their synthesis is necessary. As is well known in the art, primary amino groups present on some of the bases, however, should be protected before the base is coupled with the carbohydrate. The useful amino-protecting groups are used, including silyl groups such as having been discussed as well as such typical groups as *t*-butoxycarbonyl, benzyloxycarbonyl, 4-methoxybenzyloxycarbonyl, 4-nitrobenzyloxycarbonyl, formyl, or acetyl.

Converting the keto oxygen atoms on the bases to the enol form, in order to make the base more highly aromatic and allowing a more ready attack of the base by the carbohydrate is advisable. Enolization is provided most conveniently by producing the silyl protecting groups. The usual silyl protecting groups, as discussed above, may be used for this purpose.

In essence, the compounds embraced by formula 1 are prepared by standard coupling reactions of the 2-desoxy-2,2-difluorocarbohydrate with the appropriately protected base.

The reaction between the protected carbohydrate and the base preferably is performed neat an elevated temperature in the range of from about 50° to about 200°. Use of relatively high-boiling solvents for the reaction, such as dimethylformamide, dimethylacetamide, or hexamethylphosphoramide, however, is possible. If the coupling reaction is carried out at elevated pressures to avoid distillation of a low-boiling solvent, any convenient inert reaction solvent can be used.

The coupling reaction may be done at low temperatures if a reaction initiator, such as a trifluoromethanesulfonyloxysilane, is used. The usual inert reaction solvent, as discussed above, may be used at temperatures in the range of from about ambient temperature to about 100°.

The final step of the reaction sequence is the removal of the protecting groups. Most silyl protecting groups are cleaved easily by contact with water or an alcohol. The *t*-butyldimethylsilyl protecting group requires acid conditions, such as contact with gaseous hydrogen halide, for its removal.

Acyl protecting groups are removed by simple hydrolysis with strong or moderately strong bases, such as alkali metal hydroxides, at temperatures from about ambient temperature to about 100°. At least one equivalent amount of base is needed for each protecting group. Such hydrolyses conveniently are carried out in hydroxylic solvents, especially aqueous alkanols. The reactions also may be carried out, however, in any convenient solvent, such as polyols including ethylene glycol, ethers such as tetrahydrofuran, ketones such as acetone and methyl ethyl ketone, and other polar solvents such as dimethylsulfoxide. The cleavage of acyl protecting groups may also be performed with other bases including, for example, sodium methoxide, potassium *t*-butoxide, hydrazine, hydroxylamine, ammonia, alkali metal amides and secondary amines such as diethylamine. The acyl protecting groups also can be removed with acid catalysts, such as methanesulfonic acid, hydrochloric acid, hydrobromic acid, sulfuric acid, or with acidic ion exchange resins. Carrying out such hydrolyses at a relatively high temperature such as the reflux temperature of the mixture is preferred but temperatures as low as ambient may be used when particularly strong acids are used.

The removal of protecting groups which are ethers is carried out by known methods, for example, with ethanethiol and aluminum chloride.

None of the reaction steps require unusual excesses of the reactants. As usual in organic syntheses, use of a moderate excess, in the range of 1.05$^{x}$ to 2$^{x}$ is advisable.

The antitumor compounds provided by this invention are capable of forming pharmaceutically-acceptable addition salts. Such salts are to be construed as included within the scope of this invention and may include hydrobromide, hydrochloride, mono-, di-or triphosphate esters and sodium salts of such phosphates, sulfate, the sodium, potassium, lithium or ammonium salts, as well as others well-known to those skilled in the art. "Pharmaceutically-acceptable salts" are those salts useful in the chemotherapy of warm-blooded animals.

Having described the general methods for the preparation of the compounds of Formula I the following examples, obtained from the published British patent application 2136425A, are provided to further illustrate the preparation of the required intermediates and final compounds embraced by Formula I.

## EXAMPLE I

Ethyl 2,2-difluoro-3-hydroxy-3-(2,2-dimethyldioxolan-4-yl)propionate

To 10.2 g. of activated zinc was added a small portion of a solution consisting of 31.8 g. of ethyl bromodifluoroacetate and 22.6 g. of 4-formyl-2,2-dimethyldioxolane in 53 ml. of tetrahydrofuran and 53 ml. of diethyl ether. Care was taken to exclude water from the reaction mixture. The solution began to reflux as soon as the first addition to the activated zinc was made. The remainder of the solution was added dropwise at a rate to maintain gentle reflux throughout the addition time of 30 minutes. The mixture was then stirred under gentle reflux for 30 minutes more. The reaction mixture was poured into 200 ml. of 1N hydrochloric acid and 200 g of ice, and the mixture was stirred until all of the ice had melted. The aqueous mixture was then extracted four times with 70 ml. portions of diethyl ether, and the organic layers were combined and washed with 50 ml. of saturated aqueous sodium chloride and with 50 ml. of saturated aqueous sodium bicarbonate, dried over magnesium sulfate and evaporated under vacuum to obtain 26 g. of light yellow oil. The crude product was chromatographed on a 1000 g. silica gel column, eluting with chloroform containing 0.5% methanol to separate the major 3-R-hydroxy product from the minor 3-S-hydroxy product. The ratio of amounts of the two products was about 3:1: the minor product came off the column first.

Evaporation of the fractions containing the 3-R-hydroxy product provided 12.6 g. of the product in substantially pure form.

## EXAMPLE 2

2-desoxy-2,2-difluororibonic acid $\gamma$-lactone

Fifty g. of the 3-R-hydroxy product obtained from a synthesis similar to that of Example 1 above was dissolved in 500 ml. of methanol and 250 ml. of water, and 250 g. of Dowex 50W-X12 resin was added. The mixture was stirred at ambient temperature for 4 days, and the mixture was then filtered through a pad of diatomaceous earth filter aid. The filtrate was evaporated to dryness under vacuum to obtain 33.0 g. of the desired product.

## EXAMPLE 3

3,5-bis(*t*-butyldimethylsilyloxy)-2-desoxy-2,2-difluororibonic acid γ-lactone

To 13 g. of the product obtained in Example 2 above was added 60 ml. of dichloromethane, 22.5 ml. of 2,6-lutidine and 48.2 ml. of trifluoromethylsulfonyloxy--butyldimethylsilane under nitrogen with mild cooling to keep the temperature below 25°. Within 15 minutes after combining the reagents, the reaction became quite exothermic and the mixture became thin and easily stirred. The mixture was stirred overnight. The mixture was diluted with 150 ml. of ethyl acetate, and was washed successively with 40 ml. of 1N hydrochloric acid, 40 ml. of saturated aqueous sodium chloride. It was then dried over magnesium sulfate and evaporated to dryness under vacuum to obtain 32.1 g. of crude product, which was chromatographed on 260 g. of 100-mesh silica gel, eluting with 10:1 chloroform: diethyl ether. The fractions which contain the desired product were combined and evaporated under vacuum to obtain 7.8 g. of pure product.

## EXAMPLE 4

3,5-bis(*t*-butyldimethylsilyl)-2-desoxy-2,2-difluororibose

A 10.3 g. portion of 3,5-bis-(*t*-butyldimethylsilyloxy)-2-desoxy-2,2-difluororibonic acid γ-lactone obtained from preparations similar to that of Preparation 3 above, was dissolved in 120 ml. of anhydrous toluene and cooled to -84°. To the solution was added 26 g. of diisobutyl-aluminum hydride, added over a period of 20 minutes with constant stirring. The reaction mixture was held below -65° at all times. Two hours after the first addition of hydride, the reaction mixture was quenched with methanol at -20°, additional cold methanol was added until no more gassing occurred. The mixture was then allowed to warm slowly to ambient temperature, and was washed with 100 ml. of 0.1N hydrochloric acid. The aqueous layer was then washed with 100 ml. of diethyl ether, and then three times with 50 ml. portions of diethyl ether. The organic layers were combined, washed with 100 ml. of saturated aqueous sodium bicarbonate, dried over magnesium sulfate and evaporated under vacuum to dryness to obtain 8.2 g. of the desired product in crude form.

This material may be chromatographed, if necessary, on silica gel (25 g. silica/1 g. of crude product) using 100% dichloromethane for elution.

## EXAMPLE 5

3,5-bis(*t*-butyldimethylsilyloxy)-1-methanesulfonyloxy-2-desoxy-2,2-difluororibose

A 0.5 g. portion of 3,5-bis - (*t*-butyldimethylsilyloxy)-2-desoxy-2,2-difluororibose was dissolved in 5 ml. of anhydrous dichloromethane and 0.17 g. of triethylamine. To the solution was added with mild cooling, 0.11 ml. of methanesulfonyl chloride. After three hours of stirring under nitrogen at about 25°, the mixture was evaporated under vacuum, and the residue was taken up in 10 ml. of ethyl acetate. The solution was extracted with 3 ml. of saturated aqueous sodium bicarbonate, and then successively with 3 ml. of 1N hydrochloric acid, 3 ml. of water and 3 ml. of saturated aqueous sodium chloride. The organic solution was then dried over sodium sulfate and concentrated under vacuum to obtain 0.59 g. of the desired product.

## EXAMPLE 6

1-(5-methyl-2,4-dioxo-1H,3H-pyrimidin-1-yl)-2-desoxy-2,2-difluororibose

Under nitrogen, to 2.59 g. of 3,5-bis-(*t*-butyldimethylsiloxy)-1-methanesulfonyloxy-2-desoxy-2,2-difluororibose was added 1.60 g. of 5-methyl-2,4-bis(trimethylsilyloxy)pyrimidine and 145 ml. of dry 1,2-dichloroethane. To this mixture was added 1.45 g. of trifluoromethanesulfonyloxytrimethylsilane, and the clear solution was stirred under reflux for about 2-3 hours. The reaction was then cooled to ambient temperature and 1.35 ml. of methanol were added and the suspension was stirred for 30 minutes. The precipitate was filtered and the filtrate was reduced to one-half its volume under vacuum and then diluted with an equal volume of dichloromethane. The solution was washed with saturated aqueous sodium bicarbonate and then with saturated aqueous sodium chloride, and dried over anhydrous sodium sulfate. The solution was filtered and the filtrate was saturated with anhydrous hydrogen bromide. The reaction mixture was stirred for 30 minutes and was then concentrated under vacuum. The residue was dissolved in methanol and the solution was evaporated to dryness under vacuum. The residue was dissolved in water and the solution was extracted twice with diethyl ether. The water layer was then

evaporated to dryness. The residue was taken up in ethanol and evaporated repeatedly to azeotrope off all water. One g. of crude product was obtained, and was chromatographed on 30 g. of Woelm silica gel (70-150 mesh), eluting with ethyl acetate to yield 0.76 g. of desired product. It was further purified by recrystallization from ethyl acetate to obtain 0.37 g. of white crystalline product.

EXAMPLE 7

1-(4-amino-2-oxo-1H-pyrimidin-1-yl)-2-desoxy-2,2-difluororibose

Under a nitrogen atmosphere, to 5.0 g. of 3,5-bis(*t*-butyldimethylsiloxy)-1-methanesulfonyloxy-2-desoxy-2,2-difluororibose in 100 ml. of dry 1,2-dichloroethane was added 4.68 g. of bis-trimethylsilyl-N-acetylcytosine followed by 3.96 g. of trifluoromethanesulonyloxytrimethylsilane. The solution is refluxed for 3 to 15 hours. The reaction is cooled to room temperature, 2.0 ml. of methanol are added and the suspension is stirred for about 30 minutes. The precipitate is filtered and the filtrate is concentrated in vacuo, to dryness. The residue is dissolved in methylene chloride, saturated with anhydrous HBr and stirred at room temperature about 45 minutes. The mixture is concentrated to dryness in vacuo, taken up into saturated methanolic ammonia and stirred about 15 hours at room temperature. The solution is concentrated to dryness in vacuo, triturated with $H_2O$ and the aqueous soluble portion is applied to a reversed phase HPLC column producing 100 mg. of the desired product when eluted with water.

EXAMPLE 8

1-(4-amino-5-iodo-2-oxo-1H-pyrimidin-1-yl)-2-desoxy-2,2-difluororibose

Under a nitrogen atmosphere, to 1.99 g. of 3,5-bis(*t*-butyldimethylsiloxy)-1-methanesulfonyloxy-2-desoxy-2,2-difluororibose in 35 ml of dry 1,2-dichloroethane was added 2.08 g. of tris-trimethylsilyl-5-iodocytosine followed by 1.11 g. of trifluoromethanesulfonyloxytrimethylsilane. The solution is refluxed for 3 to 15 hours. The reaction is cooled to room temperature, 5.0 ml. of methanol are added and the suspension is stirred for about 30 minutes. The precipitate is filtered and the filtrate is concentrated in vacuo, to dryness. The residue is dissolved in methylene chloride, saturated with anhydrous HBr and stirred at room temperature for about 45 minutes. The mixture is concentrated to dryness in vacuo. The residue is triturated with $H_2O$, neutralized with $NaHCO_3$ and separated on a reversed phase HPLC column using $H_2O$:$CH_3OH$(9:1) as eluent to yield 26 mg. of the desired product.

**Claims**

1. The use of a nucleoside of the formula

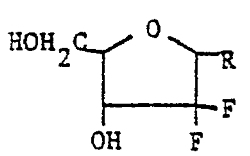

including their tautomeric and enantiomorphic forms, or a pharmaceutically acceptable salt thereof wherein R is selected from the group of base moieties consisting of

5-R'-2,4-dioxo-1H,3H-pyrimidin-1-yl (1a),

4-amino-5-R'-2-oxo-1H-pyrimidin-1-yl-(1b),

4-$R^2$-5-($R^3$-2-vinyl)-2-oxo-1H-pyrimidin-1-yl (1c),

2-amino-6-oxo-1H,9H-purin-9-yl (1d),

6-amino-9H-purin-9-yl (1e),

4-amino-2-oxo-1H-1,3,5-triazin-1-yl (1f),

6-amino-4-oxo-1,5-dihydro-4H-imidazo[4,5-c]-pyridin-1-yl (1g),

1,5-dihydro-5-methyl-3-amino-1,4,5,6,8-pentaazaacenaphthylen-1-yl (1h),

4-amino-5-carboxamido-7H-pyrrol[2,3-d]pyrimidin-7-yl (1i),

2-amino-6-thione-1,9-dihydro-6H-purin-9-yl (1j),

4-amino-2-oxo-3,6-dihydro-1H-1,3,5-triazin-1-yl (1k),

wherein R' is hydrogen, methyl, iodo, bromo, fluoro or chloro,

R² is hydroxy or amino,

R³ is bromo, chloro or iodo

for preparing a pharmaceutical composition for the treatment of neoplastic diseases.

2. Embodiment according to Claim 1 wherein the nucleoside is 2'-desoxy-2',2'-difluorocytidine.

3. Embodiment according to Claims 1 or 2 for preparing a pharmaceutical composition which additionally contains an ornithine decarboxylase inhibitor.

4. Embodiment according to Claim 3 wherein the ornithine decarboxylase inhibitor is α-difluoromethyl ornithine, α-monofluoromethyl dehydroornithine, (2 R, 5R)-6-heptyne-2,5-diamine, or the methyl or ethyl ester of monofluoromethyl dehydro ornithine.

5. Embodiment according to Claim 4 wherein the nucleoside 2'-desoxy-2',2'-difluorocytidine is used together with α-difluoromethyl ornithine.

6. A nucleoside compound of the formula

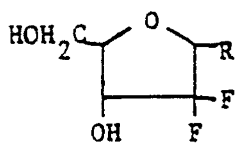

including their tautomeric and enantiomorphic forms, and the pharmaceutically acceptable salts thereof wherein R is

4-amino-2-oxo-1H-1,3,5-triazin-1-yl (1f),

6-amino-4-oxo-1,5-dihydro-4H-imidazo[4,5-c]pyridin-1-yl (1g),

1,5-dihydro-5-methyl-3-amino-1,4,5,6,8-pentaazaacenaphythylen-1-yl (1h),

4-amino-5-carboxamido-7H-pyrrol[2,3-d]pyrimidin-7-yl (1i),

2-amino-6-thione-1,9-dihydro-6H-purin-9-yl (1j),

4-amino-2-oxo-3,6-dihydro-1H-1,3,5-triazin-1-yl (1d).